(19) Europäisches Patentamt
European Patent Office
Office européen des brevets

(11) Veröffentlichungsnummer: **0 008 627**
**B1**

# (12) EUROPÄISCHE PATENTSCHRIFT

(45) Veröffentlichungstag der Patentschrift: **03.10.84**

(51) Int. Cl.³: **C 07 D 239/90,** C 09 B 29/52, C 09 B 35/03

(21) Anmeldenummer: **79102211.4**

(22) Anmeldetag: **02.07.79**

(54) **Chinazolin-Derivate, sowie Azofarbstoffe, die sich von den Chinazolin-Derivaten ableiten und deren Verwendung als Pigmente.**

(30) Priorität: **12.07.78 DE 2830555**

(43) Veröffentlichungstag der Anmeldung: **19.03.80 Patentblatt 80/6**

(45) Bekanntmachung des Hinweises auf die Patenterteilung: **03.10.84 Patentblatt 84/40**

(84) Benannte Vertragsstaaten: **CH DE FR GB**

(56) Entgegenhaltungen:
**Chemical Abstracts, Band 70, Nr. 15, 14. April 1969,Zusammenfassung Nr. 68304c, Seite 381, Columbus Ohio, USA, L. BEREZOWSKI et al.: "Synthesis and reactions of 2-(carbethoxy-methyl)-4-hydroxyquinazoline and its derivatives"**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Rolf, Meinhard, Dr., Walter-Flex-Strasse 26, D-5090 Leverkusen 1 (DE)**
Erfinder: **Neeff, Rütger, Dr., Berta-von-Suttner-Strasse 22, D-5090 Leverkusen 1 (DE)**
Erfinder: **Müller, Walter, Ing.-grad., Pfarrer-Klein-Strasse 3, D-5090 Leverkusen 3 (DE)**

**EP 0 008 627 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Chinazolin-Derivate der Formel

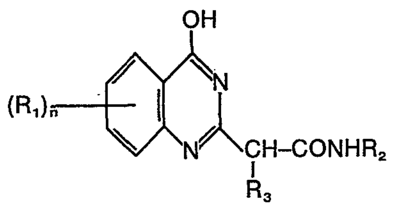

(I)

worin

n 0, 1, 2, 3 oder 4,

$R_1$ Halogen, Alkyl, Nitro, Alkylsulfonylamino, Alkylcarbonylamino, $C_1$–$C_4$-Alkoxy, Trifluormethyl, Phthalimidyl, Carboxy, Cyan, gegebenenfalls durch $C_1$–$C_4$-Alkyl, $C_1$–$C_4$-Alkylphenyl, $C_1$–$C_4$-Alkoxyphenyl, Fluorphenyl, Chlorphenyl, Bromphenyl, Phthalimidylpheny, Nitrophenyl und Benzyl substituiertes Carbamoyl oder Sulfamoyl, gegebenenfalls im Benzolkern durch Chlor, $C_1$–$C_4$-Alkyl, $C_1$–$C_4$-Alkoxy oder Nitro substituiertes Benzoylamino, gegebenenfalls durch $C_1$–$C_4$-Alkyl, $C_1$–$C_4$-Alkoxy, Fluor, Chlor, Brom oder Nitro substituiertes Phenylamino, gegebenenfalls durch $C_1$–$C_4$-Alkyl, $C_1$–$C_4$-Alkoxy, Fluor, Chlor, Brom oder Nitro substituiertes Phenylsulfonylamino,

$R_2$ Phenyl, das 1 bis 5 Substituenten aus der Reihe Chlor, Brom, Fluor, $C_1$–$C_4$-Alkyl, $C_1$–$C_4$-Alkoxy, Nitro, Trifluormethyl, Cyan, Carboxy, $C_1$–$C_4$-Alkoxycarbonyl, Phthalimidyl, Alkylsulfonylamino, Alkylcarbonylamino, gegebenenfalls durch $C_1$–$C_4$-Alkyl, $C_1$–$C_4$-Alkylphenyl, $C_1$–$C_4$-Alkoxyphenyl, Fluorphenyl, Chlorphenyl, Bromphenyl, Phthalimidylphenyl, Nitrophenyl und Benzyl substituiertes Carbamoyl oder Sulfamoyl gegebenenfalls im Benzolkern durch Chlor, $C_1$–$C_4$-Alkyl, $C_1$–$C_4$-Alkoxy oder Nitro substituiertes Benzoylamino, gegebenenfalls durch $C_1$–$C_4$-Alkyl, $C_1$–$C_4$-Alkoxy, Fluor, Chlor, Brom oder Nitro substituiertes Phenylamino, gegebenenfalls durch $C_1$–$C_4$-Alkyl, $C_1$–$C_4$-Alkoxy, Fluor, Chlor, Brom oder Nitro substituiertes Phenylsulfonylamino tragen kann,

einen Rest der Formel

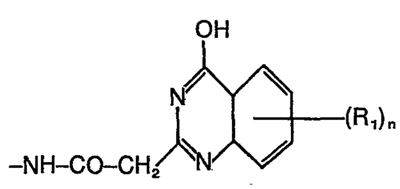

worin

$R_1$ und n die obengenannte Bedeutung haben, α-Naphthyl, β-Naphthyl, α-Anthrachinonyl, β-Anthrachinonyl, α-Pyridyl, 2-Benzthiazolyl oder 5-Benzimidazolonyl und

$R_3$ Wasserstoff oder eine Gruppe D–N=N– bezeichnen, bei dem D den Rest eines von Sulfonsäuregruppen freien aromatischen oder heteroaromatischen Amins bedeuten.

Aus Chemical Abstracts 70, 68304c (1969) sind Chinazolin-Derivate bekannt, die in 2-Stellung eine Carbalkoxymethylgruppe tragen. Die DE-OS 2 644 265 beschreibt Methylen-bis-chinazolinon-Verbindungen. Aus Chemical Abstracts 78, 17618r (1973) sind Chinazolin-Derivate bekannt, die in 2-Stellung eine am N-Atom unsubstituierte Carbamoylmethylgruppe aufweisen.

Vorzugsweise geeignete Substituenten $R_1$ sind Halogen, insbesondere Chlor und Brom, Alkyl, insbesondere $C_1$–$C_4$-Alkyl, Nitro, Alkylsulfonylamino, insbesondere $C_1$–$C_4$-Alkylsulfonylamino und Alkylcarbonylamino, insbesondere ($C_1$–$C_4$-Alkyl-)carbonylamino.

n steht bevorzugt für 0, 1 oder 2.

Die Verbindungen der Formel (I), bei denen $R_3$ = H ist, werden hergestellt durch Umsetzung von aromatischen Aminen der Formel

$$R_2-NH_2$$

wobei

$R_2$ die obengenannte Bedeutung hat,

mit funktionellen Derivaten der Chinazolinonyl-Essigsäure.

Als Derivate der Chinazolinonyl-Essigsäure kommen insbesondere die Halogenide, vor allem das Chlorid, die Alkylester der Formel (III)

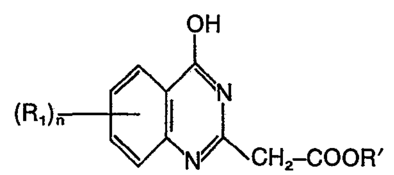

(III)

wobei

R′ für $C_1$–$C_4$-Alkyl steht und

$R_1$ sowie n die obengenannte Bedeutung haben,

die Iminoalkylester sowie das Nitril in Frage.

Die Reaktion wird bei 120 bis 220 °C in Substanz oder in einem indifferenten organischen Lösungsmittel wie o-Dichlorbenzol, 1.2.4-Trichlorbenzol, Ethylenglykoldimethylester, Ethylenglykoldiethylether, Xylol oder Nitrobenzol durchgeführt.

Die Verbindungen der Formel (I), bei denen $R_3$ = H ist, eignen sich als Kupplungskomponenten für die Herstellung von Azofarbstoffen, insbesondere für die Herstellung von Azopigmenten.

Ein weiterer Gegenstand der Erfindung sind daher Azofarbstoffe der Formel

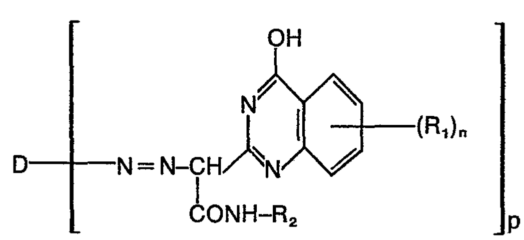

(IV)

worin

D den Rest eines von Sulfonsäuregruppen freien aromatischen oder heteroaromatischen Amins und

p eine ganze Zahl, vorzugsweise 1 oder 2 bedeuten und

$R_1$, $R_2$ und n die obengenannte Bedeutung haben.

Geeignete Diazokomponenten sind beispielsweise
Anilin, 2-Methylanilin, 2,4-Dimethylanilin,
2-Nitranilin, 3-Nitranilin, 4-Nitranilin,
2,4-Dinitranilin, 2-Chlor-4-nitranilin,
4-Chlor-2-nitranilin, 2-Chlor-5-nitranilin,
2-Nitro-4-methylanilin,
2-Methyl-4-nitranilin,
2-Methyl-5-nitranilin,
4-Methoxy-2-nitranilin,
2-Cyan-4-nitranilin, 2-Brom-4-nitranilin,
2-Nitro-4-methylsulfonylanilin,
2-Nitro-4-ethylsulfonylanilin,
2-Chloranilin, 4-Chloranilin,
2,4-Dichloranilin, 2,5-Dichloranilin,
2,6-Dichloranilin, 3,4-Dichloranilin,
3,5-Dichloranilin, 2,4,5-Trichloranilin,
2,4,6-Trichloranilin, 2-Cyan-5-chloranilin,
2-Methyl-4-chloranilin,
2-Methyl-5-chloranilin,
2,4-Dichlor-5-ethylanilin,
2,5-Dichlor-4-methylanilin,
2-Chlor-4-methylsulfonylanilin,
2-Cyan-5-chloranilin,
2,4-Dichlor-5-methoxyanilin,
2-Chlor-5-trifluormethylanilin,
4-Chlor-2-trifluormethylanilin,
3,5-Bis-trifluormethylanilin,
2,4-Dimethoxyanilin, 2,5-Dimethoxyanilin,
2,5-Diethoxyanilin,
2,4-Dimethoxy-5-chloranilin,
2,5-Dimethoxy-4-chloranilin,
2-Methoxy-5-methylanilin,
4-Methoxy-2-methylanilin,
2-Methoxy-5-methyl-4-chloranilin,
2-Methoxy-4-nitranilin,
4-Methoxy-2-nitranilin,
2-Methoxy-5-nitranilin,
2,5-Dimethoxy-4-nitranilin,
2-Methoxy-5-methyl-4-nitranilin,
2-Methoxy-5-chlor-4-nitranilin,
2-Methoxy-5-ethylsulfonylanilin,
2-Methoxy-5-phenylsulfonylanilin,
2-Methoxy-5-benzylsulfonylanilin,
2-Methoxy-4-chloranilin,
2-Ethoxy-4-chloranilin,
2-Methoxy-5-chloranilin,
2-Ethoxy-5-chloranilin,
2-Methoxy-4,5-dichloranilin,
2-Amino-5-chlordiphenylether,
2-Amino-4,4'-dichlordiphenylether,
2-Amino-4,6-dichlordiphenylether,
4-Amino-5-methoxybenzolsulfonsäure-(4-nitrophenyl)-ester,
5-Acetylamino-2-nitranilin,
5-Acetylamino-2-chlor-5-methylanilin,
4-Acetylamino-2,5-dichloranilin,
5-Acetylamino-2,4-dichloranilin,
4-Benzoylamino-2-methyl-5-methoxyanilin,
5-Benzoylamino-2-chloranilin,
4-Benzoylamino-2-chlor-5-methoxyanilin,
2-Amino-benzoesäuremethylester,
2-Aminobenzoesäureethylester,
2-Amino-benzoesäureisobutylester,
4-Chlor-2-amino-benzoesäuremethylester,
5-Chlor-2-aminobenzoesäuremethylester,
6-Chlor-2-aminobenzoesäuremethylester,
3,5-Dichlor-2-aminobenzoesäuremethylester,
4,6-Dichlor-2-aminobenzoesäuremethylester,
5-Brom-2-aminobenzoesäuremethylester,
4-Nitro-2-amino-benzoesäuremethylester,
5-Nitro-2-aminobenzoesäuremethylester,
4-Methyl-2-aminobenzoesäuremethylester,
5-Methyl-2-aminobenzoesäuremethylester,
6-Methyl-2-aminobenzoesäuremethylester,
4-Trifluormethyl-2-aminobenzoesäuremethylester,
4-Methoxy-2-aminobenzoesäuremethylester,
4-Methoxy-3-aminobenzoesäurephenylester,
4-Carbamoyl-2-aminobenzoesäuremethylester,
4-Acetylamino-2-aminobenzoesäuremethylester,
4-Benzoylamino-2-aminobenzoesäuremethylester,
4-(2,5-Dichlorbenzoylamino)-2-aminobenzoesäuremethylester,
4-Sulfamoyl-2-aminobenzoesäuremethylester,
2-Aminonaphthalin-3-carbonsäuremethylester,
4-Methyl-3-aminobenzoesäuremethylester,
1-Aminobenzol-2,5-dicarbonsäuredimethylester,
1-Aminobenzol-3,5-dicarbonsäuredimethylester,
2-Aminobenzoesäureamid,
4-Aminobenzoesäureamid,
4-Chlor-3-aminobenzoesäureamid,
4,6-Dichlor-3-aminobenzoesäureamid,
3-Amino-4-methoxy-benzoesäureamid,
3-Amino-4-methoxybenzoesäurephenylamid,
3-Amino-4-methylbenzoesäuremethylamid,
3-Amino-4-methylbenzoesäure-(2,4-dimethylphenyl)-amid,
1-Aminobenzol-3,5-dicarbonsäureamid,
3-Amino-4-methylbenzoesäure-(2,5-dichlorphenyl)-amid,
3-Amino-4-methoxycarbonylbenzoesäureamid,
3-Amino-4-methoxycarbonylbenzoesäurephenylamid,
3-Amino-4-methoxycarbonylbenzoesäure-(2,5-dichlorphenyl)-amid,
3-Amino-4-methoxybenzolsulfonsäuremethylamid,
3-Amino-4-methoxybenzolsulfonsäurediethylamid,
2,5-Dimethoxy-4-aminobenzolsulfonsäuremethylamid,
2-Methyl-5-methoxy-4-aminobenzolsulfonsäuremethylamid,
3-Amino-4-methylbenzolsulfonsäurephenylamid,
4-Amino-2,5-dimethoxybenzolsulfonsäuremethylamid,
4-Amino-2-methyl-5-methoxybenzolsulfonsäuremethylamid,
2-Chlor-1-aminonaphthalin,

1-Amino-2-methoxynaphthalin,
1-Amino-4-nitronaphthalin,
2-Amino-5-nitronaphthalin,
2-Aminothiazol, 2-Amino-4-methylthiazol,
2-Amino-5-chlorthiazol,
2-Amino-5-nitrothiazol,
2-Amino-4-methylthiazol-5-carbonsäuremethylester,
2-Amino-4-methylthiazol-5-carbonsäuredimethylamid,
2-Aminobenzthiazol,
2-Amino-6-methylbenzthiazol,
2-Amino-5-methoxybenzthiazol,
2-Amino-6-methoxybenzthiazol,
2-Amino-6-chlorbenzthiazol,
2-Amino-6-methylsulfonylbenzthiazol,
6-Methyl-2-(4-aminophenyl)-benzthiazol,
5-Amino-3-phenyl-1,2,4-thiadiazol,
2-Amino-4-methylcarbostyril,
6-Amino-4-methyl-2-chlorcarbostyril,
3-Amino-4-methoxybenzoxazol,
6-Amino-2,4-dihydroxychinazolin,
1-Aminoanthrachinon,
2-Aminoanthrachinon,
1-Amino-2-chloranthrachinon,
1-Amino-4-chloranthrachinon,
1-Amino-5-chloranthrachinon,
1-Amino-6-chloranthrachinon,
1-Amino-6(7)-chloranthrachinon (Gemisch),
1-Amino-5,8-dichloranthrachinon,
1-Amino-2-bromanthrachinon,
1-Amino-2,4-dibromanthrachinon,
1-Amino-6,7-dichloranthrachinon,
1-Amino-6-fluoranthrachinon,
1-Amino-7-fluoranthrachinon,
1-Amino-6,7-difluoranthrachinon,
2-Amino-1-chloranthrachinon,
2-Amino-3-chloranthrachinon,
2-Amino-3-bromanthrachinon,
1-Amino-4-nitroanthrachinon,
1-Amino-5-nitroanthrachinon,
1-Amino-2-methylanthrachinon,
1-Amino-2-methyl-4-chloranthrachinon,
1-Amino-2-methyl-4-bromanthrachinon,
1-Aminoanthrachinon-2-carbonsäure,
1-Aminoanthrachinon-2-carbonsäureamid,
1-Aminoanthrachinon-2-carbonsäuremethylester,
1-Amino-4-nitroanthrachinon-2-carbonsäure,
1-Amino-2-acetylanthrachinon,
1-Amino-4-acetylaminoanthrachinon,
1-Amino-5-acetylaminoanthrachinon,
1-Amino-5-benzoylaminoanthrachinon,
1-Amino-4-benzoylaminoanthrachinon,
1-Amino-8-benzoylaminoanthrachinon,
1-Amino-4-hydroxyanthrachinon,
1-Amino-5-hydroxyanthrachinon,
1-Amino-4-methoxyanthrachinon,
1-Amino-2-methoxy-4-hydroxyanthrachinon,
1-Amino-4-methylaminoanthrachinon,
1-Amino-4-benzylaminoanthrachinon,
1-Amino-4-cyclohexylaminoanthrachinon,
1-Amino-4-anilinoanthrachinon,
1-Amino-2-brom-4-methylmercaptoanthrachinon,
1-Amino-4-(4-methylphenyl),
(sulfonylamino)-2-phenylthioanthrachinon,
1-Amino-6-methylmercaptoanthrachinon,
2-Phenyl-6-amino-4,5-phthaloylbenzimidazol,
6-Chlor-2-amino-3,4-phthaloylacridon,
7-Chlor-2-amino-3,4-phthaloylacridon,
5-Chlor-8-amino-3,4-phthaloylacridon,
3-Aminobenzanthron, 5-Aminopyrazolanthron,
4-Aminoanthrapyrimidin,
6-Aminoanthrapyrimidin,
6-Amino-3-methylanthrapyridon,
7-Amino-3-methylanthrapyridon,
1,5-Diaminoanthrachinon,
1,4-Diaminoanthrachinon,
1,8-Diaminoanthrachinon,
1,6-/1,7-Diaminoanthrachinon (Gemisch),
2,6-Diaminoanthrachinon,
1,5-Diamino-4-chloranthrachinon,
1,4-Diamino-5-nitroanthrachinon,
1,5-Diamino-2,4,6,8-tetrabromanthrachinon,
1,5-Diamino-4,8-dihydroxyanthrachinon,
1,8-Diamino-4,5-dihydroxyanthrachinon,
4,4′-Diamino-1,1′-dianthrimid,
1-Amino-2-brom-4-(4-methylphenylsulfonylamino)-anthrachinon.

Bevorzugte Diazokomponenten sind solche der Benzol- und Anthrachinonreihe.

Besonders bevorzugt sind Farbstoffe der Formel

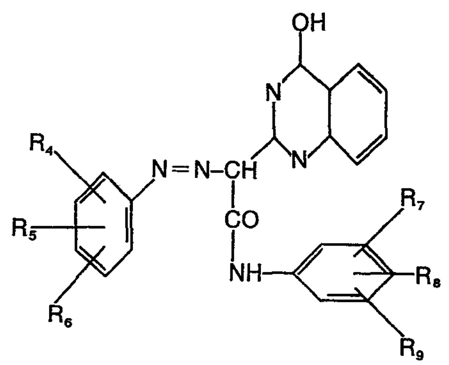

(V)

worin

$R_4$ für Wasserstoff, Halogen wie Fluor, Chlor und Brom, $C_1$–$C_4$-Alkyl, $C_1$–$C_4$-Alkoxy, Nitro, Cyan, Carboxy, $C_1$–$C_4$-Alkylsulfonyl, Trifluormethyl, $C_1$–$C_4$-Alkyl-carbonylamino, gegebenenfalls durch $C_1$–$C_4$-Alkyl, $C_1$–$C_4$-Alkoxy, Fluor, Chlor, Brom oder Nitro substituiertes Benzoylamino, $C_1$–$C_4$-Alkoxycarbonyl, gegebenenfalls durch $C_1$–$C_4$-Alkyl, Phenyl oder Benzyl mono- oder disubstituiertes Carbamoyl oder Sulfamoyl, wobei Phenyl und Benzyl durch $C_1$–$C_4$-Alkyl, $C_1$–$C_4$-Alkoxy, Fluor, Chlor, Brom und Nitro weitersubstituiert sein können, $C_1$–$C_4$-Alkylsulfonylamino, gegebenenfalls durch $C_1$–$C_4$-Alkyl, $C_1$–$C_4$-Alkoxy, Fluor, Chlor, Brom oder Nitro substituiertes Phenylsulfonylamino,

$R_5$ für Wasserstoff, Halogen wie Fluor, Chlor und Brom, $C_1$–$C_4$-Alkyl, Cyano, $C_3$–$C_4$-Alkoxy, Nitro oder Trifluormethyl,

$R_6$ für Wasserstoff, Chlor, $C_1$–$C_4$-Alkyl oder $C_1$–$C_4$-Alkoxy,

$R_7$ für Wasserstoff, Halogen wie Fluor, Chlor und Brom, $C_1$–$C_4$-Alkyl, $C_1$–$C_4$-Alkoxy, Nitro, Cyan, $C_1$–$C_4$-Alkylsulfonyl, Trifluormethyl, $C_1$–$C_4$-Alkylcarbonylamino, gegebenenfalls durch $C_1$–$C_4$-Alkyl, $C_1$–$C_4$-Alkoxy, Fluor, Chlor, Brom oder Nitro substituiertes Benzoylamino, $C_1$–$C_4$-Alkoxycarbonyl, gegebenenfalls durch $C_1$–$C_4$-Alkyl, Phenyl oder Benzyl mono- oder disubstituiertes Carbamoyl oder Sulfamoyl, wobei Phenyl und Benzyl durch $C_1$–$C_4$-Alkyl, $C_1$–$C_4$-Alkoxy, Fluor, Chlor, Brom und Nitro weitersubstituiert sein können,

$R_8$ für Wasserstoff, Halogen wie Fluor, Chlor, Brom, $C_1$–$C_4$-Alkyl, Cyano, $C_1$–$C_4$-Alkoxy, Nitro oder Trifluormethyl und

$R_9$ für Wasserstoff, Chlor, $C_1$–$C_4$-Alkyl, $C_1$–$C_4$-Alkoxy oder für einen Rest der Formel

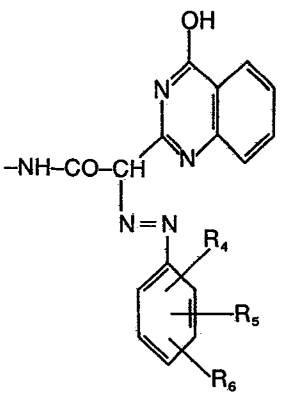

(VI)

mit den obengenannten Bedeutungen für $R_4$, $R_5$ und $R_6$ steht.

Weiterhin bevorzugt sind Farbstoffe der Formel

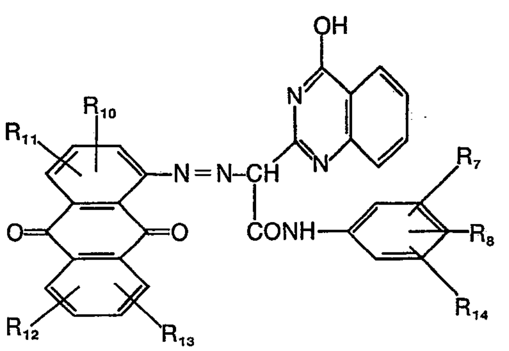

(VII)

worin

$R_7$ und $R_8$ die obengenannte Bedeutung haben und

$R_{10}$, $R_{11}$, $R_{12}$ und $R_{13}$ für Wasserstoff, Chlor, Brom, Carboxy, $C_1$–$C_4$-Alkoxycarbonyl, Carbonamid, $C_1$–$C_4$-Alkylcarbonylamino, gegebenenfalls durch 1 oder 2 Nitro oder 1 bis 5 Chlor oder Brom substituiertes Benzoylamino, $C_1$–$C_4$-Alkylsulfonylamino oder gegebenenfalls durch Methyl, Methoxy oder Chlor substituiertes Phenylsulfonylamino und

$R_{14}$ für Wasserstoff, Chlor, $C_1$–$C_4$-Alkyl, $C_1$–$C_4$-Alkoxy oder für den Rest der Formel

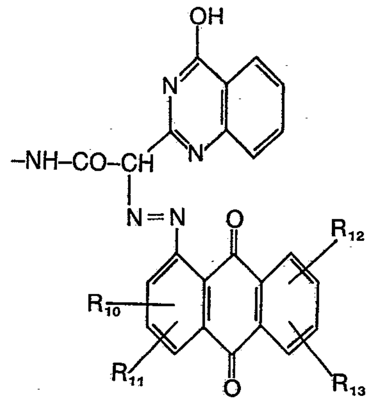

(VIII)

mit den obengenannten Bedeutungen für $R_{10}$–$R_{13}$ steht.

Die Herstellung der Azofarbstoffe IV erfolgt durch Kupplung diazotierter aromatischer Amine der Formel

$$D\text{–}(NH_2)p \qquad (IX)$$

worin

D und p die obengenannten Bedeutungen besitzen, auf die heterocyclischen Verbindungen der Formel (I), bei denen $R_3$ = H ist.

Für die Kupplungsreaktionen können mehrere Verfahren angewendet werden.

(1) Die Kupplungskomponente wird alkalisch gelöst oder suspendiert, auf einen geeigneten pH-Wert eingestellt und mit einer sauren Lösung bzw. Suspension eines Diazoniumsalzes versetzt. Man rührt bis zum Ende der Reaktion und reinigt den Farbstoff gegebenenfalls durch Erhitzen in einem organischen Lösungsmittel wie n-Butanol, Toluol, Chlorbenzol, Pyridin, Nitrobenzol, o-Dichlorbenzol, 1,2,4-Trichlorbenzol, Tetramethylensulfon, Dimethylformamid, N-Methylpyrrolidon, Ethylenglykoldimethylether oder Ethylenglykoldiethylether.

(2) Die Diazokomponente IX wird in einem organischen Lösungsmittel wie Dimethylformamid, Dimethylacetamid, Dimethylsulfoxid, Tetramethylensulfon, Tetraphenylharnstoff, N-Methylpyrrolidon, Nitrobenzol, o-Dichlorbenzol, 1,2,4-Trichlorbenzol, Ethylenglykoldimethylether, Ethylenglykoldiethylether oder Essigsäure in Gegenwart einer Säure wie Schwefelsäure, Phosphorsäure, Benzolsulfonsäure, Ethansulfonsäure, p-Toluolsulfonsäure, Naphthalin-2,6-disulfonsäure, Ameisensäure, Essigsäure, Dichloressigsäure, 2,4-Dichlorbenzoesäure, Oxalsäure, Bernsteinsäure, Maleinsäure, Weinsäure oder Terephthalsäure mit organischen Nitriten wie Methylnitrit, Ethylnitrit, iso-Amylnitrit oder vorteilhafterweise mit Nitriten von Glykolen und Glykolderivaten wie Methoxyethylnitrit. Ethoxyethylnitrit oder Alkalimetallnitriten wie Natriumnitrit diazotiert. Dann rührt man eine Suspension der Kupplungskomponente zweckmässigerweise im gleichen Lösungsmittel ein. Nach beendeter Kupplung wird das Rohprodukt durch Temperaturerhöhung auf 90 bis 200 °C in der Kupplungslösung gereinigt und durch Absaugen isoliert.

Das zweite Verfahren kann auch dahingehend variiert werden, dass man die Diazokomponente

und Kupplungskomponente im organischen Lösungsmittel vorlegt und das Alkylnitrit oder Alkalimetallnitrit zugibt, so dass Diazotierung und Kupplung gleichzeitig erfolgen. Auch bei dieser Verfahrensvariante schliesst sich zweckmässigerweise eine Temperaturbehandlung zur Reinigung des so hergestellten Pigmentes an.

Die Azofarbstoffe der Formel IV fallen in einer für Pigmente geeigneten Form an oder können durch an sich bekannte Nachbehandlungsverfahren in die geeignete Form übergeführt werden, z.B. durch Lösen oder Quellen in starken anorganischen Säuren wie Schwefelsäure und Austragen auf Eis. Die Feinverteilung kann auch durch Mahlen mit oder ohne Mahlhilfsstoffe wie anorganische Salze oder Sand, gegebenenfalls in Anwesenheit von Lösungsmitteln wie Toluol, Xylol, Dichlorbenzol oder N-Methylpyrrolidon erzielt werden. Farbstärke und Transparenz des Pigmentes können durch Variation der Nachbehandlung beeinflusst werden.

Die Pigmente der Formel IV eignen sich aufgrund ihrer Licht- und Migrationsechtheit für die verschiedensten Pigmentapplikationen. So können sie zur Herstellung von sehr echt pigmentierten Systemen, wie Mischungen mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit Zement verstanden werden. Zubereitungen sind z.B. Flushpasten mit organischen Flüssigkeiten oder Teige und Feinteige mit Wasser, Dispergiermittel und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentlacke, Dispersionsfarben für wetterfeste Überzüge und Leimfarben. Unter Druckfarben sind solche für den Papier-, Textil- und Blechdruck zu verstehen. Sie eignen sich besonders auch zur Pigmentierung von makromolekularen organischen Stoffen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polypropylen, Polyester, z.B. Polyethylenterephthalat, Polyamide, Superpolyamide, Polymerisate und Mischpolymerisate aus Acrylestern, Methacrylestern, Acrylnitril, Acrylamid, Butadien, Styrol sowie Polyurethane und Polycarbonate. Die mit den beanspruchten Produkten pigmentierten Stoffe können in beliebiger Form vorliegen.

Die erfindungsgemässen Pigmente IV sind weiterhin ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig und in plastischen Massen gut verteilbar.

Überraschenderweise zeigt das erfindungsgemässe Pigment der Formel

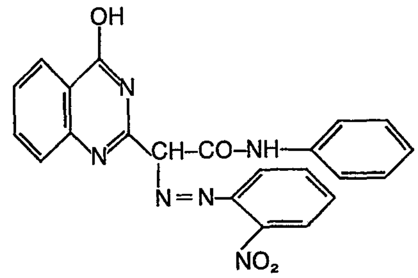

das durch Kupplung von diazotiertem o-Nitroanilin auf die Kupplungskomponente der Formel

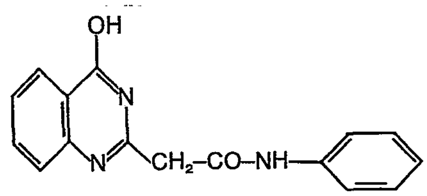

hergestellt worden ist, gegenüber dem aus Chemical Abstracts 78, 17618r (1973) bekannten, strukturell nächstliegenden Farbstoff eine deutlich verbesserte Überlackierechtheit.

Beispiel 1

Zu 700 ml 1,2-Dichlorbenzol gibt man 40 g Anilin und 96 g des Chinazolinonylessigesters der Formel

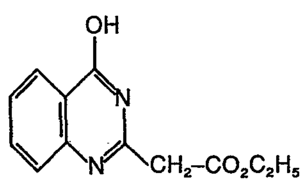

(X)

und rührt 6 Stunden bei 170 bis 180 °C. Entstehender Alkohol wird dabei abdestilliert. Nach Abkühlen saugt man ab, wäscht mit 1,2-Dichlorbenzol und Methanol nach und trocknet bei 80 °C.

Man erhält 113 g (98% d. Th.) der Verbindung der Formel

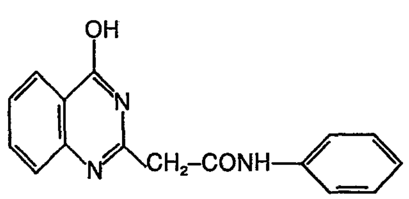

(XI)

als kristallines, weisses Pulver mit einem Schmelzpunkt oberhalb 260 °C.

| | | | |
|---|---|---|---|
| Berechnet: | C 68,6 | H 4,7 | N 15,1 |
| Gefunden: | C 68,5 | H 4,8 | N 15,1 |

Weitere Kupplungskomponenten mit den in der folgenden Tabelle angegebenen Strukturen werden nach dem in Beispiel 1 beschriebenen Verfahren erhalten, wenn man anstelle von Anilin substituierte Aniline und anstelle des Chinazolinonylessigesters (X) substituierte Chinazolinonylessigester einsetzt.

Tabelle 1

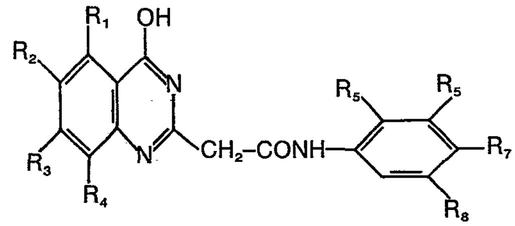

| Beispiel | $R_1$ | $R_2$ | $R_3$ | $R_4$ | $R_5$ | $R_6$ | $R_7$ | $R_8$ |
|---|---|---|---|---|---|---|---|---|
| 2 | H | H | H | H | Cl | H | H | H |
| 3 | H | H | H | H | $CH_3$ | H | H | H |
| 4 | H | H | H | H | $OCH_3$ | H | H | H |
| 5 | H | H | H | H | H | H | Cl | H |
| 6 | H | H | H | H | Cl | H | Cl | H |
| 7 | H | H | H | H | H | H | $CH_3$ | H |
| 8 | H | H | H | H | Cl | H | H | Cl |
| 9 | H | H | H | H | H | H | $NHCOCH_3$ | H |
| 10 | H | $NO_2$ | H | H | $CH_3$ | H | H | H |
| 11 | H | Br | H | H | Cl | H | H | H |
| 12 | H | Cl | H | Cl | Cl | H | $NO_2$ | H |
| 13 | Cl | Cl | Cl | Cl | $OCH_3$ | H | H | H |
| 14 | H | H | $CH_3$ | H | H | H | CN | H |
| 15 | H | H | $OCH_3$ | H | H | $CF_3$ | H | H |
| 16 | H | H | $CF_3$ | H | H | H | $NHCOCH_3$ | H |
| 17 | H | H | $SO_2CH_3$ | H | H | F | H | H |
| 18 | H | H | 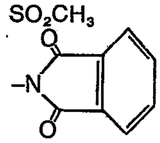 | H | H | H | $CH_3$ | H |
| 19 | H | H | COOH | H | $NO_2$ | H | $NHCOC_6H_5$ | H |
| 20 | H | H | CN | H | H | H | $CONH_2$ | H |
| 21 | H | H | $CONHC_6H_5$ | H | H | H | $SO_2CH_3$ | H |
| 22 | H | H | $SO_2N(CH_3)_2$ | H | H | H | $OC_2H_5$ | H |
| 23 | H | H | $NHCOCH_3$ | H | $CH_3$ | H | $CH_3$ | H |
| 24 | H | H | $NHCOC_6H_5$ | H | Cl | H | Cl | Cl |
| 25 | H | H | $NHC_6H_5$ | H | H | H | $SO_2NH_2$ | H |

Die Strukturen der so hergestellten Verbindungen werden durch die Elementaranalysen und die Massenspektren bestätigt.

Beispiel 26

Nach dem in Beispiel 1 genannten Verfahren werden bei Verwendung von 20 g p-Phenylendiamin anstelle von Anilin 68 g (77% der Theorie) der Kupplungskomponente der Formel

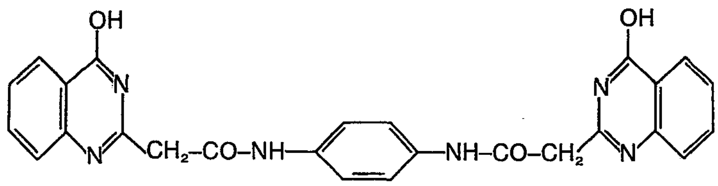

(XII)

als weisses Pulver mit einem Schmelzpunkt > 300 °C erhalten. Die Struktur wird durch Elementaranalyse und Massenspektren bestätigt.

Beispiel 27

12 g nach Beispiel 7 erhaltener Kupplungskomponente werden in 100 ml 3%iger Natronlauge 60 Minuten bei 20 °C verrührt. Anschliessend stellt man durch Zugabe von Eisessig auf pH 5, kühlt auf 5 °C ab und tropft eine filtrierte Diazoniumsalzlösung aus 7,2 g 2-Nitro-4-choranilin ein. Nach 3stündigem Nachrühren wird abgesaugt, neutral gewaschen und getrocknet.

Man erhält 19,3 g (97% der Theorie) des Pigmentes der Formel

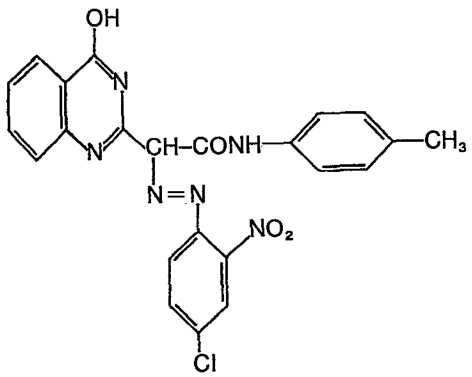

(XIII)

in gelben Nadeln mit einem Schmelzpunkt > 300 °C.

Beispiel 28

In 300 ml Dimethylformamid gibt man 20 ml konz. $H_2SO_4$ und 22 g 2-Nitro-4-chloranilin. Nach Abkühlen auf 10 °C wird durch Zugabe einer Lösung von 17 g Amylnitrit in 30 ml Dimethylformamid diazotiert und eine Suspension von 50 g der nach Beispiel 5 erhaltenen Verbindung in 100 ml Dimethylformamid eingerührt. Man rührt 3 Stunden bei 25 °C nach, saugt ab, wäscht mit Dimethylformamid und Methanol und trocknet.

Man erhält 54 g (95% d. Th.) des Gelbpigmentes der Formel

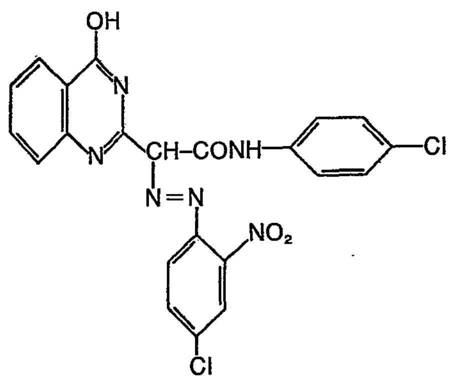

(XIV)

mit einem Schmelzpunkt > 300 °C.

Beispiel 29

In ein Gemisch aus 200 ml Nitrobenzol und 30 ml 85%iger Ameisensäure gibt man 15 g 1-Aminoanthrachinon und 23 g der nach Beispiel 2 erhaltenen Verbindung. Man rührt 30 Minuten bei Raumtemperatur und tropft dann eine Lösung von 6 g $NaNO_2$ in 10 ml Wasser ein. Nach 2 Stunden wird im Vakuum trockengeheizt, 1 Stunde bei 150 °C getempert und bei 50 °C abgesaugt. Man wäscht mit Nitrobenzol, Methanol und Wasser nach und trocknet.

Man erhält 29,5 g (80% der Theorie) des Gelbpigmentes der Formel

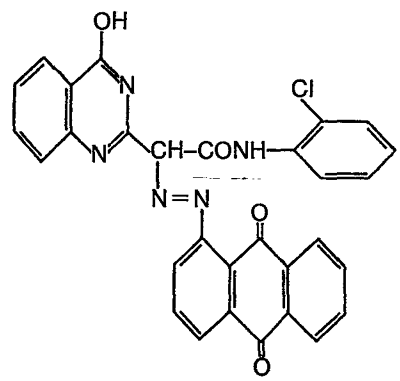

(XV)

mit einem Schmelzpunkt 300 °C.

Weitere Pigmente mit den in der folgenden Tabelle 2 angegebenen Farbtönen werden nach dem in Beispiel 16 genannten Verfahren erhalten, wenn man anstelle des 2-Nitro-4-chloranilins die in der zweiten Spalte angegebenen Diazakomponenten und anstelle der Kupplungskomponente aus Beispiel 5 die in der dritten Spalte angegebenen Kupplungskomponenten verwendet.

Tabelle 2

| Beispiel | Diazokomponente | Kupplungskomponente | Farbton |
|---|---|---|---|
| 30 | 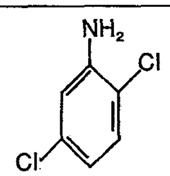 | Beispiel 2 | grünst. gelb |
| 31 | 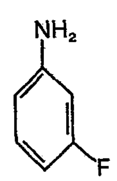 | Beispiel 5 | grünst. gelb |
| 32 | 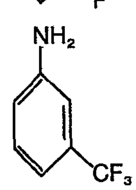 | Beispiel 4 | gelb |

| Beispiel | Diazokomponente | Kupplungskomponente | Farbton |
|---|---|---|---|
| 33 | 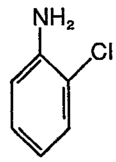 | Beispiel 14 | gelb |
| 34 | 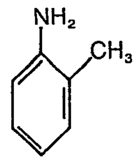 | Beispiel 23 | gelb |
| 35 | 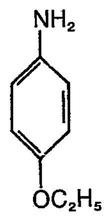 | Beispiel 18 | gelb |
| 36 | 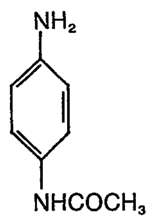 | Beispiel 16 | gelb |
| 37 | 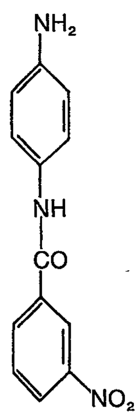 | Beispiel 3 | gelb |
| 38 | 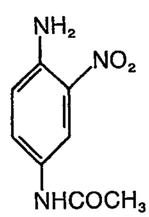 | Beispiel 14 | gelb |
| 39 | 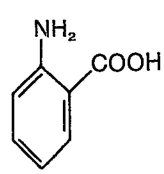 | Beispiel 22 | grünst. gelb |
| 40 | 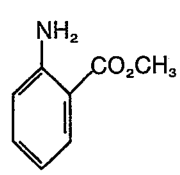 | Beispiel 24 | gelb |

| Beispiel | Diazokomponente | Kupplungskomponente | Farbton |
|---|---|---|---|
| 41 | 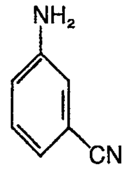 | Beispiel 21 | grünst. gelb |
| 42 | 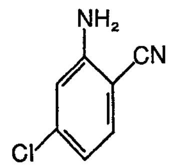 | Beispiel 9 | grünst. gelb |
| 43 | 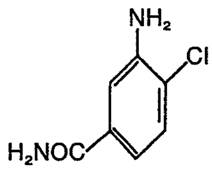 | Beispiel 25 | gelb |
| 44 | 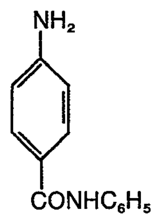 | Beispiel 17 | gelb |
| 45 | 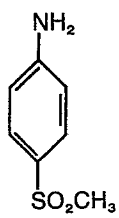 | Beispiel 20 | gelb |
| 46 | 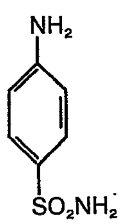 | Beispiel 9 | gelb |
| 47 | 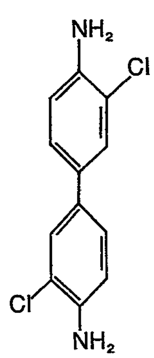 | Beispiel 3 | gelb |
| 48 | 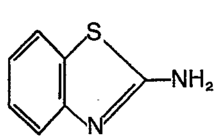 | Beispiel 10 | orange |

| Beispiel | Diazokomponente | Kupplungskomponente | Farbton |
|---|---|---|---|
| 49 | 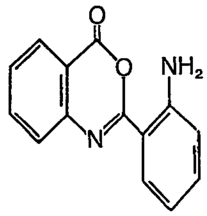 | Beispiel 5 | gelb |
| 50 | 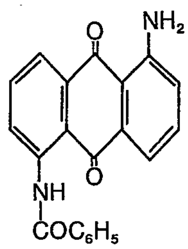 | Beispiel 6 | orange |
| 51 | 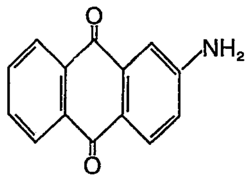 | Beispiel 5 | gelb |
| 52 | 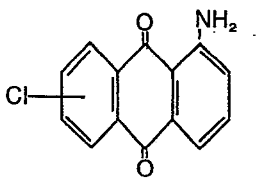 | Beispiel 2 | gelb |
| 53 | 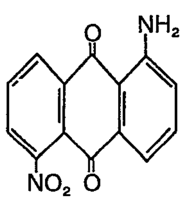 | Beispiel 7 | gelb |
| 54 | 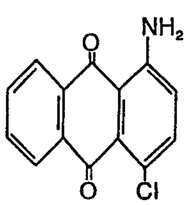 | Beispiel 9 | gelb |
| 55 | 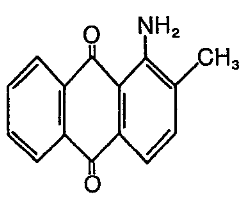 | Beispiel 2 | gelb |
| 56 | 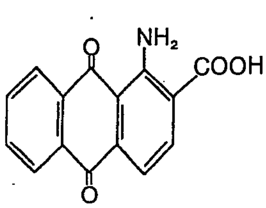 | Beispiel 2 | gelb |

| Beispiel | Diazokomponente | Kupplungskomponente | Farbton |
|---|---|---|---|
| 57 | 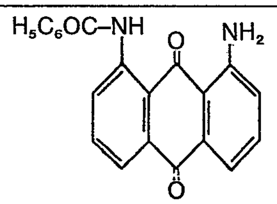 | Beispiel 3 | rotst. gelb |
| 58 | 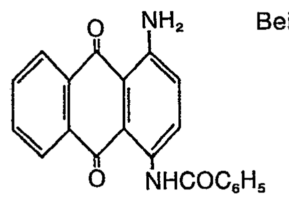 | Beispiel 4 | braun |
| 59 | 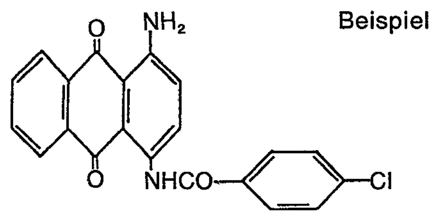 | Beispiel 4 | braun |
| 60 | 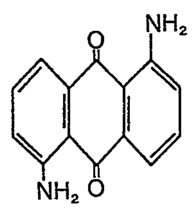 | Beispiel 7 | orange |
| 61 | 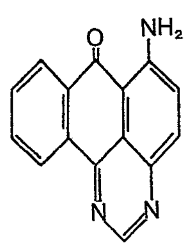 | Beispiel 2 | gelb |

Beispiel 62

a) 8 g des nach Beispiel 28 erhaltenen feinverteilten Pigmentes werden mit einem Einbrennlack aus 25 g Kokosölalkydharz (40% Kokosöl), 10 g Melaminharz, 50 g Toluol und 7 g Glykolmonomethylether auf einer automatischen Hoover-Muller-Anreibmaschine angerieben. Man trägt die Mischung auf die zu lackierende Unterlage auf, härtet den Lack durch Einbrennen bei 130 °C und erhält gelbe Lackierungen sehr guter Überlackierechtheit und hervorragender Licht- und Wetterechtheit.

Pigmentierte Einbrennlacke gleicher Echtheiten erhält man, wenn man 15 bis 25 g des angegebenen Alkydharzes oder eines Alkydharzes auf Basis von Baumwollsaatöl, Ricinenöl, Ricinusöl oder synthetischen Fettsäuren verwendet und statt der angegebenen Melaminharzmenge 10 bis 15 g des erwähnten Melaminharzes oder eines Kondensationsproduktes von Formaldehyd mit Harnstoff oder mit Benzoguanamin einsetzt.

b) Reibt man statt der angegebenen Pigmentmenge 1 bis 10 g einer Mischung von Titandioxid (Rutiltyp) mit dem in Beispiel 62a angegebenen Pigment im Verhältnis 0,5–50 : 1 in den im Beispiel 62a angegebenen Lack ein, erhält man bei gleicher Weiterverarbeitung Lackierungen gleicher Echtheiten und mit steigendem Titandioxidgehalt nach weiss verschobenem gelben Farbton.

Beispiel 63

In 100 g eines Nitrocelluloselackes, der aus 44 g Collodiumwolle (niedrigviskos, 35%ig butanolfeucht), 5 g Dibutylphthalat, 40 g Ethylacetat, 20 g Toluol, 4 g n-Butanol und 10 g Glykolmonomethylether besteht, werden 6 g feinverteiltes Pigment gemäss Beispiel 28 eingerieben. Nach Verstreichen und Trocknen erhält man gelbe Lackierungen hervorragender Licht- und Überlackierechtheit. Zu gleichen Ergebnissen kommt man bei Verwendung von Nitrolacken mit 10 bis 15 g Nitrocellulosegehalt, 5 bis 10 g Weichmachergehalt und 70 bis 85 g Lösungsmittelgemisch unter bevorzugter Verwendung von aliphatischen Estern wie Ethylacetat, Butylacetat und Aromaten wie Toluol und Xylol und kleineren Anteilen aliphatischer Ether wie Glykolether und Alkohole wie Butanol. Unter Weichmachern können z.B. verstanden werden: Phthalsäureester wie Dioctylphthalat, Dibutylphthalat, Ester der Phosphorsäure, Ricinusöl allein oder in Kombination mit ölmodifizierten Alkydharzen.

Lackierungen mit ähnlichen Echtheitseigenschaften erhält man bei Verwendung von anderen physikalisch trocknenden Sprit-, Zapon- und Nitrolacken, von lufttrocknenden Öl-, Kunstharz- und Nitrokombinationslacken, ofen- und lufttrocknenden Epoxidharzlacken, gegebenenfalls in Kombination mit Harnstoff-, Melamin-, Alkyd- oder Phenolharzen.

Beispiel 64

5 g in feine Verteilung gebrachtes Pigment gemäss Beispiel 28 werden in 100 g eines paraffinfrei trocknenden ungesättigten Polyesterharzes in einer Porzellankugelmühle angerieben. Mit der Anreibung werden 10 g Styrol, 59% Melamin-Formaldehyd-Harz und 1 g einer Paste aus 40 g Cyclohexanonperoxid und 60% Dibutylphthalat gut verrührt und schliesslich 4 g Trocknerlösung (10%iges Kobaltnaphthenat in Testbenzin) und 1 g Siliconöllösung (1%ig in Xylol) beigemischt. Man trägt die Mischung auf grundiertes Holz auf, und erhält eine hochglänzende, wasserfeste und wetterechte gelbe Lackierung von hervorragender Lichtechtheit.

Verwendet man statt des Reaktionslackes auf Basis ungesättigter Polyesterharze aminhärtende Epoxidharzlacke mit Dipropylendiamin als Aminokomponente, erhält man gelbe Lackierungen hervorragender Wetter- und Ausblühechtheit.

Beispiel 65

100 g einer 65%igen Lösung eines aliphatischen Polyesters mit ca. 8% freien Hydroxylgruppen in Glykolmonoethyletheracetat werden mit 5 g des nach Beispiel 29 erhaltenen Pigmentes angerieben und sodann mit 44 g einer 67%igen Lösung des Umsetzungsproduktes von 1 Mol Trimethylolpropan mit 3 Mol Toluylen-diisocyanat gut vermischt. Ohne Beeinträchtigung der Tropfzeit ergeben sich nach Auftragen des Gemisches und Reaktion der Komponenten hochglänzende gelbe Polyurethanlackierungen hervorragender Ausblüh-, Licht- und Wetterechtheit.

Pigmentierungen ähnlicher Echtheiten erhält man bei Anwendung anderer Zweikomponentenlacke auf Basis von aromatischen oder aliphatischen Isocyanaten und hydroxylgruppenhaltigen Polyethern oder Polyestern, sowie mit feuchtigkeitstrocknenden, Polyharnstofflackierungen ergebenden Polyisocyanatlacken.

Beispiel 66

5 g eines Feinteiges, erhalten durch Kneten von 50 g des nach Beispiel 24 erhaltenen Pigmentes mit 15 g eines Arylpolyglykolether-Emulgators und 35 g Wasser werden mit 10 g Schwerspat als Füllstoff, 10 g Titandioxid (Rutiltyp) als Weisspigment und 40 g einer wässrigen Dispersionsfarbe, enthaltend ca. 50% Polyvinylacetat, gemischt. Man verstreicht die Farbe und erhält nach Trocknen gelbe Anstriche sehr guter Kalk- und Zementechtheit sowie hervorragender Wetter- und Lichtechtheit.

Der durch Kneten erhaltene Feinteig eignet sich gleichermassen zum Pigmentieren klarer Polyvinylacetat-Dispersionsfarben, für Dispersionsfarben, die Mischpolymerisate aus Styrol und Maleinsäuren als Bindemittel enthalten, sowie Dispersionsfarben auf Basis von Polyvinalpropionat, Polymethacrylat oder Butadienstyrol.

Beispiel 67

10 g des in Beispiel 66 erwähnten Pigment-Teigs werden mit einer Mischung aus 5 g Kreide und 5 g 20%iger Leimlösung vermischt. Man erhält eine gelbe Tapetenstreichfarbe, mit der man Überzüge hervorragender Lichtechtheit erzielt. Zur Herstellung des Pigment-Teiges können auch andere nichtionogene Emulgatoren wie die Umsetzungsprodukte von Nonylphenyol mit Ethylenoxid oder ionogene Netzmittel, wie die Natriumsalze von Alkylarylsulfonsäure, z.B. der Dinaphthylmethandisulfonsäure, Natriumsalze von substituierten Sulfofettsäureestern und Natriumsalze von Paraffinsulfonsäuren in Kombination mit Alkylpolyglykolethern verwendet werden.

Beispiel 68

Eine Mischung aus 65 g Polyvinylchlorid, 35 g Diisooctylphthalat, 2 g Dibutylzinnmercaptid, 0,5 g Titandioxid und 0,5 g des Pigmentes von Beispiel 28 wird auf einem Mischwalzwerk bei 165 °C eingefärbt. Man erhält eine intensiv gelbe Masse, die zur Herstellung von Folien oder Formkörpern dienen kann. Die Färbung zeichnet sich durch hervorragende Licht- und sehr gute Weichmacherechtheit aus.

Beispiel 69

0,2 g Pigment nach Beispiel 28 werden mit 100 g Polyethylen-, Polypropylen- oder Polystyrolgranulat gemischt. Die Mischung kann entweder bei 220 bis 280 °C direkt in einer Spritzgussmaschine verspritzt, oder in einer Strangpresse zu gefärbten Stäben bzw. auf dem Mischwalzwerk zu gefärbten Fellen verarbeitet werden. Die Stäbe bzw. Felle werden gegebenenfalls granuliert und in einer Spritzgussmaschine verspritzt.

Die gelben Formlinge besitzen sehr gute Licht- und Migrationsechtheit. In ähnlicher Weise können bei 280 bis 300 °C, gegebenenfalls unter Stickstoffatmosphäre, synthetische Polyamide aus Caprolactam oder Adipinsäure und Hexamethylendiamin oder die Kondensate aus Terephthalsäure und Ethylenglykol gefärbt werden.

Beispiel 70

1 g Pigment nach Beispiel 28, 10 g Titandioxid (Rutiltyp) und 100 g eines in Pulverform vorliegenden Mischpolymerisates auf Basis von Acrylnitril-Butadien-Styrol werden gemischt und auf einem Walzwerk bei 140 bis 180 °C eingefärbt. Man erhält ein gelbes Fell, das granuliert und in einer Spritzgussmaschine bei 200 bis 250 °C verspritzt wird. Man erhält gelbe Formlinge sehr guter Licht- und Migrationsechtheit sowie ausgezeichneter Hitzebeständigkeit.

Auf ähnliche Weise, jedoch bei Temperaturen von 180 bis 220 °C und ohne Zusatz von Titandioxid werden Kunststoffe auf Basis von Celluloseacetat, Cellulosebutyrat und deren Gemische mit ähnlichen Echtheiten gefärbt.

Beispiel 71

0,2 g Pigment nach Beispiel 28 werden in feinverteilter Form mit 100 g eines Kunststoffes auf Polycarbonat-Basis in einem Extruder oder in einer Knetschnecke bei 250 bis 280 °C gemischt und zu Granulat verarbeitet. Man erhält ein gelbes, transparentes Granulat hervorragender Lichtechtheit und Hitzebeständigkeit.

Beispiel 72

90 g eines schwach verzweigten Polypropylenglykols mit einem Molekulargewicht von 2500 und einer Hydroxylzahl von 56, 0,25 g Endoethylenpiperazin, 0,3 g Zinn-(II)-octoat, 1,0 g eines Polyethersiloxans, 3,5 g Wasser, 12,0 g einer Anreibung von 10 g Pigment nach Beispiel 29 in 50 g des angegebenen Polypropylenglykols werden gut miteinander gemischt und anschliessend mit 45 g Toluylendiisocyanat (80% 2,4- und 20% 2,6-Isomeres) innig gemischt und in eine Form gegossen. Die Mischung trübt sich nach 6 Sekunden und die Schaumstoffbildung erfolgt. Nach 70 Sekunden hat sich ein intensiv gelber, weicher Polyurethanschaumstoff gebildet, dessen Pigmentierung hervorragende Lichtechtheit aufweist.

Beispiel 73

90 g eines schwach verzweigten Polyesters aus Adipinsäure, Diethylenglykol und Trimethylolpropan mit einem Molekulargewicht von 2000 und einer Hydroxylzahl von 60 werden mit folgenden Komponenten vermischt: 1,2 g Dimethylbenzylamin, 2,5 g Natrium-Ricinusölsulfat, 2,0 g eines oxethylierten, benzylierten Oxydiphenyls, 1,75 g Wasser, 12 g einer Paste, hergestellt durch Anreiben von 10 g des Pigmentes nach Beispiel 28 in 50 g des oben angegebenen Polyesters. Nach der Mischung werden unter Rühren 40 g Toluylendiisocyanat (65% 2,4- und 35% 2,6-Isomeres) eingerührt und die Mischung in eine Form gegossen und verschäumt. Nach 60 Sekunden hat sich ein gelber, weicher Polyurethanschaumstoff gebildet, dessen Einfärbung sich durch sehr gute Lichtechtheiten auszeichnet.

Beispiel 74

Mit einer Druckfarbe, hergestellt durch Anreiben von 35 g Pigment nach Beispiel 28 und 65 g Leinöl und Zugabe von 1 g Sicoativ (Co-Naphthenat, 50%ig in Testbenzin) werden gelbe Offset-Drucke hoher Brillanz und Farbstärke und sehr guter Licht- und Lackierechtheit erhalten. Verwendung dieser Druckfarbe in Buch-, Licht-, Stein- oder Stahlstichdruck führt zu gelben Drucken ähnlicher Echtheiten. Verwendet man das Pigment zur Färbung von Blechdruck- oder niedrigviskosen Tiefdruckfarben oder Drucktinten, erhält man gelbe Drucke ähnlicher Echtheiten.

Beispiel 75

Aus 10 g des in Beispiel 66 angegebenen Pigment-Feinteiges. 100 g Traganth 3%ig, 100 g einer wässrigen 50%igen Eialbuminlösung und 25 g eines nichtionogenen Netzmittels wird eine Druckpaste bereitet. Man bedruckt ein Textilfasergewebe, dämpft bei 100 °C und erhält einen gelben Druck, der sich durch verzügliche Echtheiten, insbesondere Lichtechtheiten auszeichnet. Im Druckansatz können anstelle des Traganths und Eialbumins weitere, für das Fixieren auf der Faser verwendbare Bindemittel, beispielsweise solche auf Kunstharzbasis, Britishgum oder Celluloseglykolat verwendet werden.

Beispiel 76

Eine Mischung aus 100 g Crepe hell, 2,6 g Schwefel, 1 g Stearinsäure, 1 g Mercaptobenzthiazol, 0,2 g Hexamethylentetramin, 5 g Zinkoxid, 60 g Kreide und 2 g Titandioxid (Anatastyp) wird auf einem Mischwalzwerk bei 50 °C mit 2 g des nach Beispiel 28 erhaltenen Pigmentes eingefärbt und dann 12 Minuten bei 140 °C vulkanisiert. Man erhält ein gelbes Vulkanisat sehr guter Lichtechtheit.

Beispiel 77

100 g einer 20%igen wässrigen Paste des Pigmentes nach Beispiel 28 beispielsweise hergestellt durch Auflösen des Farbstoffes in 96%iger Schwefelsäure, Austragen auf Eis, Filtrieren und Neutralwaschen mit Wasser, werden 22,5 l einer wässrigen, ungefähr 9%igen Viskoselösung im Rührwerk zugesetzt. Die gefärbte Masse wird 15 Minuten gerührt, anschliessend entlüftet und einen Spinn- und Entschwefelungsprozess unterworfen.

Man erhält gelbe Fäden oder Folien mit sehr guter Lichtechtheit.

Beispiel 78

10 kg Teile einer Papiermasse, enthaltend auf 100 g 4 g Cellulose, werden im Holländer während etwa 2 Stunden behandelt. Während dieser Zeit gibt man in je viertelstündigen Abständen 4 g Harzleim, dann 30 g einer etwa 15%igen Pigmentdispersion, erhalten durch Mahlen von 4,8 g des nach Beispiel 28 erhaltenen Pigmentes mit 4,8 g Dinaphthylmethandisulfonsäure und 22 g Wasser in der Kugelmühle, sodann 5 g Aluminiumsulfat zu.

Nach Fertigstellung auf der Papiermaschine erhält man ein gelbes Papier von hervorragender Lichtechtheit.

Beispiel 79

Das nach Beispiel 78 hergestellte gelb pigmentierte Papier wird mit der 55%igen Lösung eines Harnstoff-Formaldehyd-Harzes in n-Butanol getränkt und bei 140 °C eingebrannt. Man erhält ein gelbes Laminatpapier von sehr guter Migrations- und hervorragender Lichtechtheit.

Ein Laminatpapier gleicher Echtheiten erhält man durch Laminieren eines Papieres, das im Tiefdruckverfahren mit einer Druckfarbe bedruckt wurde, die den in Beispiel 66 angegebenen, gelben Pigmentfeinteig und wasserlösliche bzw. verseifbare Bindemittel enthält.

Beispiel 80

20 Teile des nach Beispiel 28 erhaltenen Pigmentes werden in 50 Teilen Dimethylformamid mit einem Dissolver vordispergiert und gegebenenfalls unter Zusatz eines Dispergierhilfsmittels und von 50 Teilen einer 10%igen Polyacrylnitrillösung in Dimethylformamid einer Mahlung in einer Perlmühle unterworfen. Die Pigmentmasse wird nach bekannten Verfahren anteilsmässig einer Spinnlösung aus Polyacrylnitril zugegeben, homogenisiert und nach einem in der Technik üblichen und bekannten Trocken- oder Nass-Spinnverfahren zu Fäden versponnen.

Die auf diese Weise erhaltenen Färbungen weisen eine sehr gute Brillanz, Reib-, Migrations-, Hitze-, Licht- und Wetterechtheit auf.

**Patentansprüche**

1. Chinazolin-Derivate der Formel

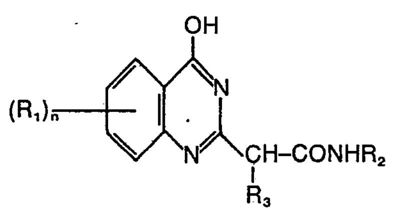

worin

n 0, 1, 2, 3 oder 4,

$R_1$ Halogen, Alkyl, Nitro, Alkylsulfonylamino, Alkylcarbonylamino, $C_1$–$C_4$-Alkoxy, Trifluormethyl, Phthalimidyl, Carboxy, Cyan, gegebenenfalls durch $C_1$–$C_4$-Alkyl, $C_1$–$C_4$-Alkylphenyl, $C_1$–$C_4$-Alkoxyphenyl, Fluorphenyl, Chlorphenyl, Bromphenyl, Phthalimidylphenyl, Nitrophenyl und Benzyl substituiertes Carbamoyl oder Sulfamoyl, gegebenenfalls im Benzolkern durch Chlor, $C_1$–$C_4$-Alkyl, $C_1$–$C_4$-Alkoxy oder Nitro substituiertes Benzoylamino, gegebenenfalls durch $C_1$–$C_4$-Alkyl, $C_1$–$C_4$-Alkoxy, Fluor, Chlor, Brom oder Nitro substituiertes Phenylamino, gegebenenfalls durch $C_1$–$C_4$-Alkyl, $C_1$–$C_4$-Alkoxy, Fluor, Chlor, Brom oder Nitro substituiertes Phenylsulfonylamino,

$R_2$ Phenyl, das 1 bis 5 Substituenten aus der Reihe Chlor, Brom, Fluor, $C_1$–$C_4$-Alkyl, $C_1$–$C_4$-Alkoxy, Nitro, Trifluormethyl, Cyan, Carboxy, $C_1$–$C_4$-Alkoxycarbonyl, Phthalimidyl, Alkylsulfonylamino, Alkylcarbonylamino, gegebenenfalls durch $C_1$–$C_4$-Alkyl, $C_1$–$C_4$-Alkylphenyl, $C_1$–$C_4$-Alkoxyphenyl, Fluorphenyl, Chlorphenyl, Bromphenyl, Phthalimidylphenyl, Nitrophenyl und Benzyl substituiertes Carbamoyl oder Sulfamoyl, gegebenenfalls im Benzolkern durch Chlor, $C_1$–$C_4$-Alkyl, $C_1$–$C_4$-Alkoxy oder Nitro substituiertes Benzoylamino, gegebenenfalls durch $C_1$–$C_4$-Alkyl, $C_1$–$C_4$-Alkoxy, Fluor, Chlor, Brom oder Nitro substituiertes Phenylamino, gegebenenfalls durch $C_1$–$C_4$-Alkyl, $C_1$–$C_4$-Alkoxy, Fluor, Chlor, Brom oder Nitro substituiertes Phenylsulfonylamino tragen kann, einen Rest der Formel

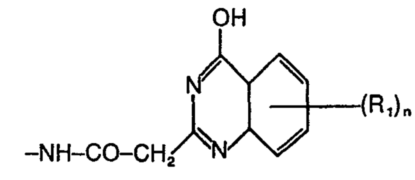

worin

$R_1$ und n die obengenannte Bedeutung haben, α-Naphthyl, β-Naphthyl, α-Anthrachinonyl, β-Anthrachinonyl, α-Pyridyl, 2-Benzthiazolyl oder 5-Benzimidazolonyl und

$R_3$ Wasserstoff oder eine Gruppe D–N=N– bezeichnen, bei dem D den Rest eines von Sulfonsäuregruppen freien aromatischen oder heteroaromatischen Amins bedeutet.

2. Chinazolin-Derivate der Formel

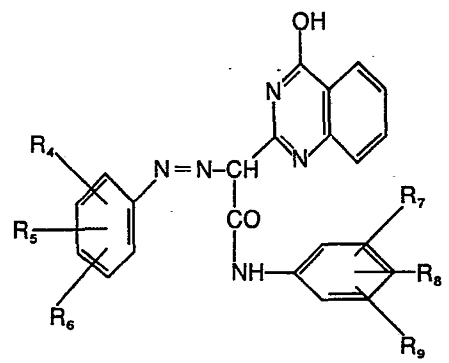

worin

$R_4$ für Wasserstoff, Halogen wie Fluor, Chlor oder Brom, $C_1$–$C_4$-Alkyl, $C_1$–$C_4$-Alkoxy, Nitro, Cyan, Carboxy, $C_1$–$C_4$-Alkylsulfonyl, Trifluormethyl, $C_1$–$C_4$-Alkylcarbonylamino, gegebenenfalls durch $C_1$–$C_4$-Alkyl, $C_1$–$C_4$-Alkoxy, Fluor, Chlor, Brom oder Nitro substituiertes Benzoylamino, $C_1$–$C_4$-Alkoxycarbonyl, gegebenenfalls durch $C_1$–$C_4$-Alkyl, Phenyl oder Benzyl mono- oder disubstituiertes Carbamoyl oder Sulfamoyl, wobei Phenyl und Benzyl durch $C_1$–$C_4$-Alkyl, $C_1$–$C_4$-Alkoxy, Fluor, Chlor, Brom und Nitro weitersubstituiert sein können, $C_1$–$C_4$-Alkylsulfonylamino, gegebenenfalls durch $C_1$–$C_4$-Alkyl, $C_1$–$C_4$-Alkoxy, Fluor, Chlor, Brom oder Nitro substituiertes Phenylsulfonylamino,

$R_5$ für Wasserstoff, Halogen wie Fluor, Chlor oder Brom, $C_1$–$C_4$-Alkyl, Cyano, $C_1$–$C_4$-Alkoxy, Nitro oder Trifluormethyl,

$R_6$ für Wasserstoff, Chlor, $C_1$–$C_4$-Alkyl oder $C_1$–$C_4$-Alkoxy,

$R_7$ für Wasserstoff, Halogen wie Fluor, Chlor und Brom, $C_1$–$C_4$-Alkyl, $C_1$–$C_4$-Alkoxy, Nitro, Cyan, $C_1$–$C_4$-Alkylsulfonyl, Trifluormethyl, $C_1$–$C_4$-(Alkyl)-carbonylamino, gegebenenfalls durch $C_1$–$C_4$-Alkyl, $C_1$–$C_4$-Alkoxy, Fluor, Chlor, Brom oder Nitro

substituiertes Benzoylamino, $C_1$–$C_4$-Alkoxycarbonyl, gegebenenfalls durch $C_1$–$C_4$-Alkyl, Phenyl oder Benzyl mono- oder disubstituiertes Carbamoyl oder Sulfamoyl, wobei Phenyl und Benzyl durch $C_1$–$C_4$-Alkyl, $C_1$–$C_4$-Alkoxy, Fluor, Chlor, Brom und Nitro substituiert sein können.

$R_8$ für Wasserstoff, Halogen wie Fluor, Chlor, Brom, $C_1$–$C_4$-Alkyl, Cyano, $C_1$–$C_4$-Alkoxy, Nitro oder Trifluormethyl und

$R_9$ für Wasserstoff, Chlor, $C_1$–$C_4$-Alkyl, $C_1$–$C_4$-Alkoxy oder für einen Rest der Formel

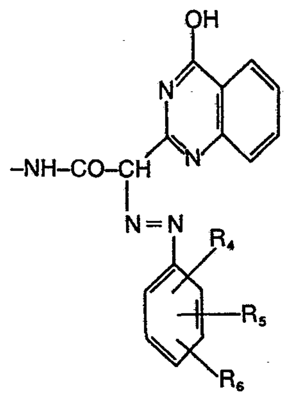

mit den obengenannten Bedeutungen für $R_4$, $R_5$ und $R_6$ steht.

3. Chinazolin-Derivate der Formel

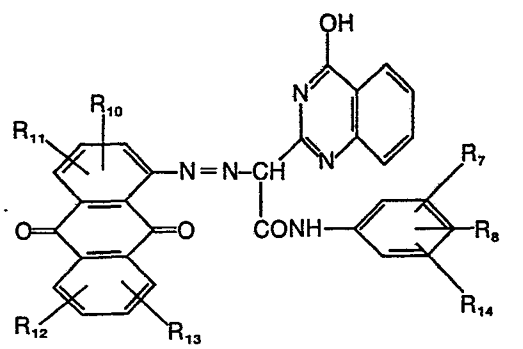

in der

$R_7$ für Wasserstoff, Halogen wie Fluor, Chlor oder Brom, $C_1$–$C_4$-Alkyl, $C_1$–$C_4$-Alkoxy, Nitro, Cyan, $C_1$–$C_4$-Alkylsulfonyl, Trifluormethyl, $C_1$–$C_4$-(Alkyl)-carbonylamino, gegebenenfalls durch $C_1$–$C_4$-Alkyl, $C_1$–$C_4$-Alkoxy, Fluor, Chlor, Brom oder Nitro substituiertes Benzoylamino, $C_1$–$C_4$-Alkoxycarbonyl, gegebenenfalls durch $C_1$–$C_4$-Alkyl, Phenyl oder Benzyl mono- oder disubstituiertes Carbamoyl oder Sulfamoyl, wobei Phenyl und Benzyl durch $C_1$–$C_4$-Alkyl, $C_1$–$C_4$-Alkoxy, Fluor, Chlor, Brom und Nitro substituiert sein können.

$R_8$ für Wasserstoff, Halogen wie Fluor, Chlor, Brom, $C_1$–$C_4$-Alkyl, Cyano, $C_1$–$C_4$-Alkoxy, Nitro oder Trifluormethyl und

$R_{10}$, $R_{11}$, $R_{12}$ und $R_{13}$ für Wasserstoff, Chlor, Brom, Carboxy, $C_1$–$C_4$-Alkoxycarbonyl, Carbonamid, $C_1$–$C_4$-(Alkyl)-carbonylamino, gegebenenfalls durch 1 oder 2 Nitro oder 1 bis 5 Chlor oder Brom substituiertes Benzoylamino, $C_1$–$C_4$-Alkylsulfonylamino oder gegebenenfalls durch Methyl, Methoxy oder Chlor substituiertes Phenylsulfonylamino und

$R_{14}$ für Wasserstoff, Chlor, $C_1$–$C_4$-Alkyl, $C_1$–$C_4$-Alkoxy oder für den Rest der Formel

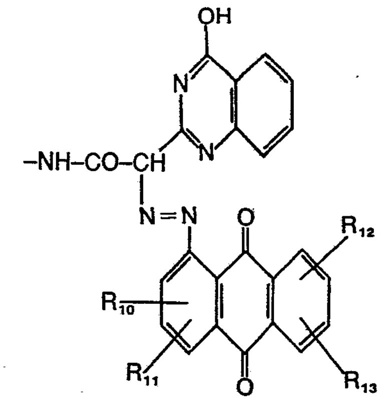

mit den obengenannten Bedeutungen für $R_{10}$ bis $R_{13}$ steht.

4. Verwendung von Azofarbstoffen gemäss den Ansprüchen 1 bis 3 als Pigmente.

**Claims**

1. Quinazoline derivatives of the formula

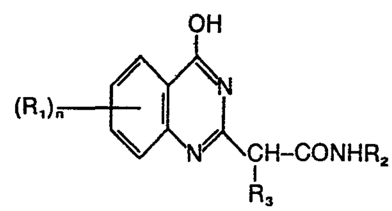

wherein

n denotes 0, 1, 2, 3 or 4,

$R_1$ denotes halogen, alkyl, nitro, alkylsulphonylamino, alkylcarbonylamino, $C_1$–$C_4$-Alkoxy, trifluormethyl, phthalimidyl, carboxyl, cyano, sulphamoyl or carbamoyl optionally substituted by $C_1$–$C_4$-alkyl, $C_1$–$C_4$-alkylphenyl, $C_1$–$C_4$-alkoxyphenyl, fluorophenyl, chlorophenyl, bromophenyl, phthalimidylphenyl, nitrophenyl or benzyl, benzoylamino optionally substituted in the benzene nucleus by chlorine, $C_1$–$C_4$-alkyl, $C_1$–$C_4$-alkoxy or nitro, phenylamino optionally substituted by $C_1$–$C_4$-alkyl, $C_1$–$C_4$-alkoxy, fluorine, chlorine, bromine or nitro, or phenylsulphonylamino optionally substituted by $C_1$–$C_4$-alkyl, $C_1$–$C_4$-alkoxy, fluorine, chlorine, bromine or nitro,

$R_2$ denotes phenyl which can carry 1 to 5 substituents from the series comprising chlorine, bromine, fluorine, $C_1$–$C_4$-alkyl, $C_1$–$C_4$-alkoxy, nitro, trifluoromethyl, cyano, carboxyl, $C_1$–$C_4$-alkoxycarbonyl, phthalimidyl, alkylsulphonylamino, alkylcarbonylamino, sulphamoyl or carbamoyl optionally substituted by $C_1$–$C_4$-alkyl, $C_1$–$C_4$-alkylphenyl, $C_1$–$C_4$-alkoxyphenyl, fluorophenyl, chlorophenyl, bromophenyl, phthalimidylphenyl, nitrophenyl or benzyl, benzoylamino optionally substituted in the benzene nucleus by chlorine, $C_1$–$C_4$-alkyl, $C_1$–$C_4$-alkoxy or nitro, phenylamino optionally substituted by $C_1$–$C_4$-alkyl, $C_1$–$C_4$-alkoxy, fluorine,

chlorine, bromine or nitro, and phenylsulphonylamino optionally substituted by $C_1$–$C_4$-alkyl, $C_1$–$C_4$-alkoxy, fluorine, chlorine, bromine or nitro, a radical of the formula

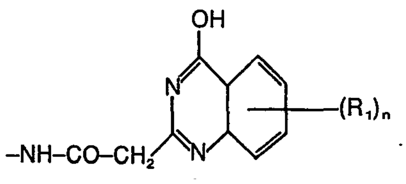

wherein

$R_1$ and n have the abovementioned meaning,

α-naphthyl, β-naphthyl, α-anthraquinonyl, β-anthraquinonyl,

α-pyridyl, 2-benzthiazolyl or 5-benzimidazolonyl and

$R_3$ denotes hydrogen or a group D–N=N– in which D denotes the radical of an aromatic or heteroaromatic amine which is free from sulphonic acid groups.

2. Quinazoline derivatives of the formula

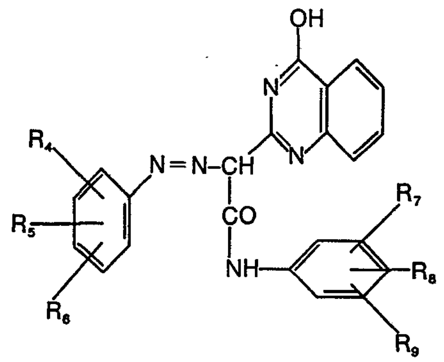

wherein

$R_4$ represents hydrogen, halogen such as fluorine, chlorine or bromine, $C_1$–$C_4$-alkyl, $C_1$–$C_4$-alkoxy, nitro, cyano, carboxyl, $C_1$–$C_4$-alkylsulphonyl, trifluoromethyl, $C_1$–$C_4$-alkylcarbonylamino, benzoylamino optionally substituted by $C_1$–$C_4$-alkyl, $C_1$–$C_4$-alkoxy, fluorine, chlorine, bromine or nitro, $C_1$–$C_4$-alkoxycarbonyl, sulphamoyl or carbamoyl optionally mono- or di-substituted by $C_1$–$C_4$-alkyl, phenyl or benzyl, it being possible for phenyl and benzyl to be further substituted by $C_1$–$C_4$-alkyl, $C_1$–$C_4$-alkoxy, fluorine, chlorine, bromine or nitro, $C_1$–$C_4$-alkylsulphonylamino, or phenylsulphonylamino optionally substituted by $C_1$–$C_4$-alkyl, $C_1$–$C_4$-alkoxy, fluorine, chlorine, bromine or nitro,

$R_5$ represents hydrogen, halogen such as fluorine, chlorine or bromine, $C_1$–$C_4$-alkyl, cyano, $C_1$–$C_4$-alkoxy, nitro or trifluoromethyl,

$R_6$ represents hydrogen, chlorine, $C_1$–$C_4$-alkyl or $C_1$–$C_4$-alkoxy,

$R_7$ represents hydrogen, halogen such as fluorine, chlorine and bromine, $C_1$–$C_4$-alkyl, $C_1$–$C_4$-alkoxy, nitro, cyano, $C_1$–$C_4$-alkylsulphonyl, trifluoromethyl, $C_1$–$C_4$-(alkyl)-carbonylamino, benzoylamino optionally substituted by $C_1$–$C_4$-alkyl, $C_1$–$C_4$-alkoxy, fluorine, chlorine, bromine or nitro, $C_1$–$C_4$-alkoxycarbonyl, sulphamoyl or carbamoyl optionally mono- or di-substituted by $C_1$–$C_4$-alkyl, phenyl or benzyl, it being possible for phenyl and benzyl to be substituted by $C_1$–$C_4$-alkyl, $C_1$–$C_4$-alkoxy, fluorine, chlorine, bromine or nitro,

$R_8$ represents hydrogen, halogen such as fluorine, chlorine, or bromine, $C_1$–$C_4$-alkyl, cyano, $C_1$–$C_4$-alkoxy, nitro or trifluoromethyl and

$R_9$ represents hydrogen, chlorine, $C_1$–$C_4$-alkyl, $C_1$–$C_4$-alkoxy or a radical of the formula

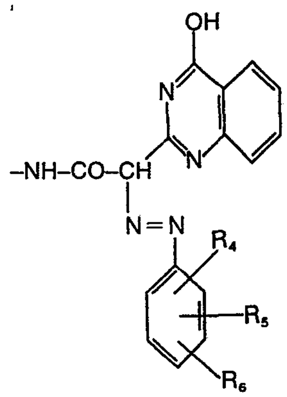

with the abovementioned meanings for $R_4$, $R_5$ and $R_6$.

3. Quinazoline derivatives of the formula

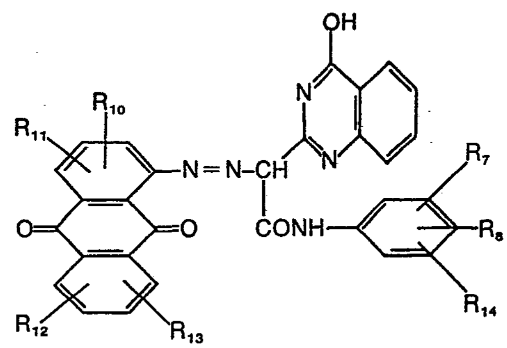

in which

$R_7$ represents hydrogen, halogen such as fluorine, chlorine or bromine, $C_1$–$C_4$-alkyl, $C_1$–$C_4$-alkoxy, nitro, cyano, $C_1$–$C_4$-alkylsulphonyl, trifluoromethyl, $C_1$–$C_4$-(alkyl)-carbonylamino, benzoylamino optionally substituted by $C_1$–$C_4$-alkyl, $C_1$–$C_4$-alkoxy, fluorine, chlorine, bromine or nitro, $C_1$–$C_4$-alkoxycarbonyl, or sulphamoyl or carbamoyl optionally mono- or di-substituted by $C_1$–$C_4$-alkyl, phenyl or benzyl, it being possible for phenyl and benzyl to be substituted by $C_1$–$C_4$-alkyl, $C_1$–$C_4$-alkoxy, fluorine, chlorine, bromine or nitro,

$R_8$ represents hydrogen, halogen such as fluorine, chlorine, or bromine, $C_1$–$C_4$-alkyl, cyano, $C_1$–$C_4$-alkoxy, nitro or trifluoromethyl and

$R_{10}$, $R_{11}$, $R_{12}$ and $R_{13}$ represent hydrogen, chlorine, bromine, carboxyl, $C_1$–$C_4$-alkoxycarbonyl, carboxamide, $C_1$–$C_4$-(alkyl)-carbonylamino, benzoylamino optionally substituted by 1 or 2 nitro or 1 to 5 chlorine or bromine, $C_1$–$C_4$-alkylsulphonylamino or phenylsulphonylamino optionally substituted by methyl, methoxy or chlorine, and

$R_{14}$ represents hydrogen, chlorine, $C_1$–$C_4$-alkyl, $C_1$–$C_4$-alkoxy or the radical or the formula

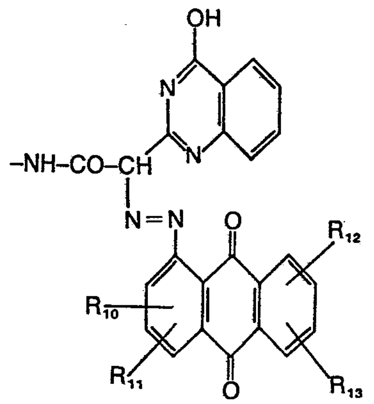

with the abovementioned meanings for $R_{10}$ to $R_{13}$.

4. Use of azo dyestuffs according to Claims 1 to 3 as pigments.

**Revendications**

1. Dérivés de quinazoline de formule

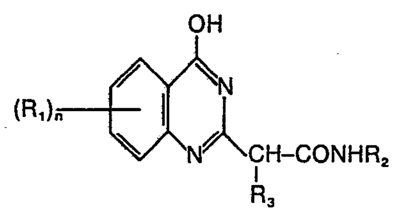

dans laquelle

n est égal à 0, 1, 2, 3 ou 4,

$R_1$ représente un halogène, un groupe alkyle, nitro, alkylsulfonylamino, alkylcarbonylamino, alkoxy en $C_1$ à $C_4$, trifluorométhyle, phtalimidyle, carboxy, cyano, carbamoyl ou sulfamoyle éventuellement substitué par un radical alkyle en $C_1$ à $C_4$, (alkyle en $C_1$ à $C_4$)-phényle, (alkoxy en $C_1$ à $C_4$)-phényle, fluorophényle, chlorophényle, bromophényle, phtalimidylphényle, nitrophényle et benzyle, un groupe benzoylamino éventuellement substitué dans le noyau benzénique par un radical chloro, alkyle en $C_1$ à $C_4$, alkoxy en $C_1$ à $C_4$ ou nitro, un groupe phénylamino éventuellement substitué par un radical alkyle en $C_1$ à $C_4$, alkoxy en $C_1$ à $C_4$, fluoro, chloro, bromo ou nitro, un groupe phénylsulfonylamino éventuellement substitué par un radical alkyle en $C_1$ à $C_4$, alkoxy en $C_1$ à $C_4$, fluoro, chloro, bromo ou nitro,

$R_2$ est un groupe phényle qui peut porter 1 à 5 substituants de la série comprenant le chlore, le brome, le fluor, un groupe alkyle en $C_1$ à $C_4$, alkoxy en $C_1$ à $C_4$, nitro, trifluorométhyle, cyano, carboxy, (alkoxy en $C_1$ à $C_4$)-carbonyle, phtalimidyle, alkylsulfonylamino, alkylcarbonylamino, carbamoyle ou sulfamoyle éventuellement substitué par un radical alkyle en $C_1$ à $C_4$, (alkyle en $C_1$ à $C_4$)-phényle, (alkoxy en $C_1$ à $C_4$)-phényle, fluorophényle, chlorophényle, bromophényle, phtalimidylphényle, nitrophényle et benzyle, un groupe benzoylamino éventuellement substitué dans le noyau benzénique par un radical chloro, alkyle en $C_1$ à $C_4$, alkoxy en $C_1$ à $C_4$ ou nitro, un groupe phénylamino éventuellement substitué par un radical alkyle en $C_1$ à $C_4$, alkoxy en $C_1$ à $C_4$ fluoro, chloro, bromo ou nitro, un groupe phénylsulfonylamino éventuellement substitué par un radical alkyle en $C_1$ à $C_4$, alkoxy en $C_1$ à $C_4$, fluoro, chloro, bromo ou nitro,

un reste de formule

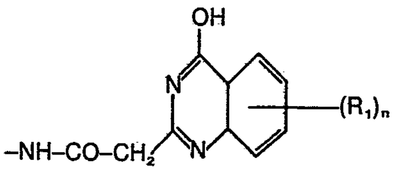

dans laquelle

$R_1$ et n ont la définition donnée ci-dessus, α-naphtyle, β-naphtyle, α-anthraquinonyle, β-anthraquinonyle, α-pyridyle, 2-benzothiazolyle ou 5-benzimidazolonyle et

$R_3$ désigne l'hydrogène ou un groupe D–N=N– dans lequel D désigne le reste d'une amine aromatique ou hétéro-aromatique dépourvue de groupes acide sulfonique.

2. Dérivés de quinazoline de formule

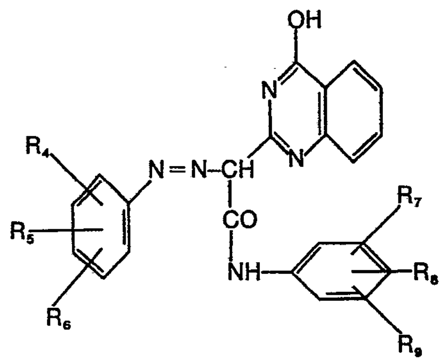

dans laquelle

$R_4$ désigne l'hydrogène, un halogène tel que le fluor, le chlore ou le brome, un groupe alkyle en $C_1$ à $C_4$, alkoxy en $C_1$ à $C_4$, nitro, cyano, carboxy, alkylsulfonyle en $C_1$ à $C_4$, trifluorométhyle, (alkyle en $C_1$ à $C_4$)-carbonylamino, un groupe benzoylamino éventuellement substitué par un radical alkyle en $C_1$ à $C_4$, alkoxy en $C_1$ à $C_4$, fluoro, chloro, bromo ou nitro, un groupe (alkoxy en $C_1$ à $C_4$)-carbonyle, un groupe carbamoyle ou sulfamoyle éventuellement monosubstitué ou disubstitué par un radical alkyle en $C_1$ à $C_4$, phényle ou benzyle, les radicaux phényle et benzyle pouvant être substitués en outre par un radical alkyle en $C_1$ à $C_4$, alkoxy en $C_1$ à $C_4$, fluoro, chloro, bromo et nitro, un groupe (alkyle en $C_1$ à $C_4$)-sulfonylamino, un groupe phénylsulfonylamino éventuellement substitué par un radical alkyle en $C_1$ à $C_4$, alkoxy en $C_1$ à $C_4$, fluoro, chloro, bromo ou nitro,

$R_5$ désigne l'hydrogène, un halogène tel que le fluor, le chlore ou le brome, un groupe alkyle en $C_1$ à $C_4$, cyano, alkoxy en $C_1$ à $C_4$, nitro ou trifluorométhyle,

$R_6$ désigne l'hydrogène, le chlore, un groupe alkyle en $C_1$ à $C_4$ ou un groupe alkoxy en $C_1$ à $C_4$,

$R_7$ désigne l'hydrogène, un halogène tel que le fluor, le chlore et le brome, un groupe alkyle en $C_1$ à $C_4$, alkoxy en $C_1$ à $C_4$, nitro, cyano, alkylsulfonyle en $C_1$ à $C_4$, trifluorométhyle, (alkyle en $C_1$ à $C_4$)-carbonylamino, un groupe benzoylamino

éventuellement substitué par un radical alkyle en $C_1$ à $C_4$, alkoxy en $C_1$ à $C_4$, fluoro, chloro, bromo ou nitro, un groupe (alkoxy en $C_1$ à $C_4$)-carbonyle, un groupe carbamoyle ou sulfamoyle éventuellement monosubstitué ou disubstitué par un radical alkyle en $C_1$ à $C_4$, phényle ou benzyle, le radical phényle et le radical benzyle pouvant être substitués par un radical alkyle en $C_1$ à $C_4$, alkoxy en $C_1$ à $C_4$, fluoro, chloro, bromo et nitro,

$R_8$ désigne l'hydrogène, un halogène tel que le fluor, le chlore, le brome, un groupe alkyle en $C_1$ à $C_4$, cyano, alkoxy en $C_1$ à $C_4$, nitro ou trifluorométhyle et

$R_9$ désigne l'hydrogène, le chlore, un groupe alkyle en $C_1$ à $C_4$, un groupe alkoxy en $C_1$ à $C_4$ ou un reste de formule

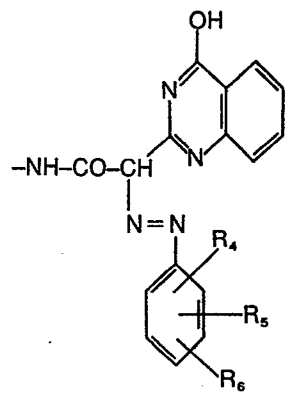

$R_4$, $R_5$ et $R_6$ ayant les définitions données ci-dessus.

3. Dérivés de quinazoline de formule

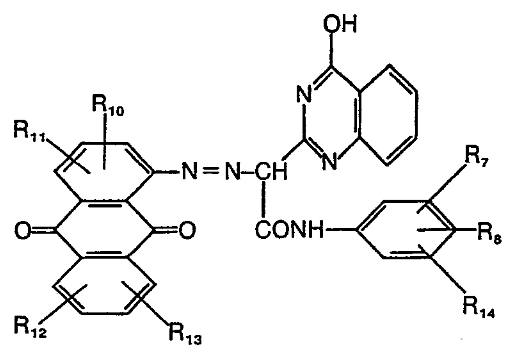

dans laquelle

$R_7$ désigne l'hydrogène, un halogène tel que le fluor, le chlore ou le brome, un groupe alkyle en $C_1$ à $C_4$, alkoxy en $C_1$ à $C_4$, nitro, cyano, alkylsulfonyle en $C_1$ à $C_4$, trifluorométhyle, (alkyle en $C_1$ à $C_4$)-carbonylamino, un groupe benzoylamino éventuellement substitué par un radical alkyle en $C_1$ à $C_4$, alkoxy en $C_1$ à $C_4$, fluoro, chloro, bromo ou nitro, un groupe (alkoxy en $C_1$ à $C_4$)-carbonyle, un groupe carbamoyle ou sulfamoyle éventuellement monosubstitué ou disubstitué par un radical alkyle en $C_1$ à $C_4$, phényle ou benzyle, les radicaux phényle et benzyle pouvant être substitués par un radical alkyle en $C_1$ à $C_4$, alkoxy en $C_1$ à $C_4$, fluoro, chloro, bromo et nitro,

$R_8$ désigne l'hydrogène, un halogène tel que le fluor, le chlore, le brome, un groupe alkyle en $C_1$ à $C_4$, cyano, alkoxy en $C_1$ à $C_4$, nitro ou trifluorométhyle et

$R_{10}$, $R_{11}$, $R_{12}$ et $R_{13}$ désignent l'hydrogène, le chlore, le brome, un groupe carboxy, (alkoxy en $C_1$ à $C_4$)-carbonyle, carboxamido, (alkyle en $C_1$ à $C_4$)-carbonylamino, un groupe benzoylamino éventuellement substitué par 1 ou 2 radicaux nitro ou par 1 à 5 atomes de chlore ou de brome, un groupe alkylsulfonylamino en $C_1$ à $C_4$ ou un groupe phénylsulfonylamino éventuellement substitué par un radical méthyle, méthoxy ou chloro et

$R_{14}$ désigne l'hydrogène, le chlore, un groupe alkyle en $C_1$ à $C_4$, alkoxy en $C_1$ à $C_4$ ou le reste de formule

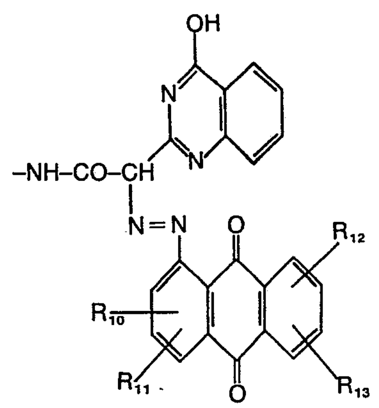

dans lequel $R_{10}$ à $R_{13}$ ont les définitions données ci-dessus.

4. Utilisation de colorants azoïques suivant les revendications 1 à 3 comme pigments.